# EUROPÄISCHE PATENTSCHRIFT

(11) **EP 1 778 307 B1**
(45) Veröffentlichungstag und Bekanntmachung des Hinweises auf die Patenterteilung: **17.03.2010**
(21) Anmeldenummer: 05784392.2
(22) Anmeldetag: 05.08.2005
(51) Int. Cl.: A61L 27/24

(54) **VERNETZTE KOLLAGENMATRIX ZUR HERSTELLUNG EINES HAUT[QUIVALENTES**
CROSS-LINKED COLLAGEN MATRIX FOR PRODUCING A SKIN EQUIVALENT
MATRICE DE COLLAGENE RETICULE SERVANT A PRODUIRE UN EQUIVALENT DE PEAU

(30) Priorität: 13.08.2004 DE 102004039537
(43) Veröffentlichungstag der Anmeldung: 02.05.2007
(73) Patentinhaber: Henkel AG & Co. KGaA, 40589 Düsseldorf (DE)
(72) Erfinder: BERND, August, 55411 Bingen-Kempten (DE); FRIESS, Wolfgang, 82393 Iffeldorf (DE); MEWES, Karsten, Rüdiger, 55128 Mainz (DE); RAUS, Martina, 61440 Oberursel (DE); BENDZ, Henriette, 81241 München (DE); KIPPENBERGER, Stefan, 60316 Frankfurt am Main (DE)
(74) Vertreter: Ioannidis, Johannes
(86) Internationale Anmeldenummer: PCT/EP2005/008478
(87) Internationale Veröffentlichungsnummer: WO 2006/018147

(56) Entgegenhaltungen:
- EP-A- 0 314 109
- EP-A- 0 702 081
- FR-A- 2 809 313
- US-A- 5 282 859
- US-A1- 2003 170 892
- PATENT ABSTRACTS OF JAPAN Bd. 2000, Nr. 08, 6. Oktober 2000 (2000-10-06) -& JP 2000 125855 A (GUNZE LTD), 9. Mai 2000 (2000-05-09)
- GEESIN J.C., BROWN L.J., LIU Z., BERG R.A.: "development of a skin model based on insoluble fibrillar collagen" JOURNAL OF BIOMEDICAL MATERIALS RESEARCH, Bd. 33, 1996, Seiten 1-8, XP008062607
- LIU J.Y., HOU L.Z., YAN W.Q.: "fabrication of tissue engineered skin equivalent" CHINESE JOURNAL OF REPARATIVE AND RECONSTRUCTIVE SURGERY, Bd. 15, Nr. 4, 2001, Seiten 235-239, XP008062613

## Beschreibung

Die vorliegende Erfindung betrifft eine vernetzte Kollagenmatrix zur Herstellung eines Hautäquivalentes, ein Dermisäquivalent, ein Epidermisäquivalent und ein Hautäquivalent auf der Basis einer solchen Kollagenmatrix sowie Verfahren zur Herstellung der Kollagenmatrix und des Dermis-, Epidermis- bzw. Hautäquivalentes.

Hauttypische Vollhautmodelle, die auch als in vitro-Hautäquivalente bezeichnet werden, können insbesondere in der Dermatologie und in der Allergologie als Testhaut verwendet werden, um Substanzen, beispielsweise potentielle Arzneimittel oder Kosmetika, oder Agenzien, wie Licht und Wärme, auf ihre pharmakologischen oder kosmetischen Wirkungen, insbesondere Reiz-, Toxizitäts- und Entzündungswirkungen, und auf ihre Verträglichkeit zu untersuchen. Ein solches System kann darüber hinaus für vielfältige immunologische, histologische und molekularbiologische Fragestellungen eingesetzt werden. Dazu zählen zum Beispiel Studien zur Wundheilung und zur Penetration und Resorption von Substanzen. Die Untersuchungen beziehungsweise Tests von Substanzen an solchen Vollhautmodellen bieten gegenüber Tierversuchen und Versuchen mit menschlichen Probanden wesentliche Vorteile, da die damit erzielten Ergebnisse reproduzierbarer sind und die Untersuchungen kostengünstiger und schneller durchgeführt werden können.

Zur Prüfung von Rohstoffen und Produkten sind in den letzten Jahren zumeist humane Zellkulturen als in vitro-Systeme verwendet worden. Eine Weiterentwicklung der Zellkulturtechnik stellen organähnliche humane Gewebe oder bioartifizielle Konstrukte und Cokultursysteme dar. Die damit gewonnenen Ergebnisse lassen sich noch besser auf den Menschen übertragen als die an Einzelzellkulturen gewonnenen Ergebnisse.

Die EP 0 197 090 B1 offenbart ein Verfahren zur Bildung eines Hautäquivalents, wobei durch Mischen einer sauren Kollagenlösung mit kontraktilen Zellen, beispielsweise Fibroblasten, ein hydratisiertes Kollagengitter hergestellt wird.

Nach Neutralisierung des pH-Wertes werden in dem Kollagengitter Kollagenfibrillen ausgefällt. Die kontraktilen Zellen lagern sich an das Kollagengitter an und bewirken dessen Kontraktion, wobei sich ein Dermisäquivalent bildet. Durch das Einbringen von Haut-Stanzbiopsien in das Kollagengitter können Keratinozyten aus den Stanzbiopsien auf der Oberfläche des Dermisäquivalentes wachsen, wobei sich ein Hautäquivalent bildet.

In der EP 0 285 474 B1 wird ein Hautäquivalent offenbart, das ein aus Kollagen und Fibroblasten erhaltenes Dermisäquivalent und ein mehrschichtiges Epidermisäquivalent umfaßt. Dabei wird das Dermisäquivalent mit einem menschlichen oder tierischen Explantat, beispielsweise einem Haarfollikel, beimpft, um das Epidermisäquivalent zu erhalten.

Die EP 0 020 753 B1 beschreibt ein Verfahren zur Bildung von Gewebe, insbesondere Hautgewebe, wobei ebenfalls Fibroblasten in ein hydratisiertes Kollagengitter eingebracht werden und sich nach Kontraktion des Kollagengitters ein Gewebe bildet. Auf dieses Gewebe können zuvor in vitro kultivierte Keratinocyten oder aus Vorhaut isolierte Keratinozyten aufgebracht werden, wobei sich ein Hautersatz bildet.

Aus der EP 0 418 035 B1 ist ein Gewebeäquivalent bekannt, das ein hydratisiertes Kollagengitter, welches mittels eines kontraktilen Agens wie Fibroblasten kontrahiert wird, und ein Kollagengel, welches mit einem permeablen Element in Kontakt steht, umfasst. Dabei wird das Gemisch aus Kollagen und kontraktilem Agens auf das Kollagengel aufgetragen, wobei durch den Kontakt zwischen Kollagengel und permeablem Element, beispielsweise einer Polycarbonatmembran, die radiale oder laterale Kontraktion des Kollagengitters unterbunden wird, so dass das Gitter nur bezüglich seiner Dicke kontrahiert. Nach Bildung des Dermisäquivalents können dann Keratinozyten ausgesät werden, wobei sich ein Hautäquivalent bildet.

Ferner offenbart das US-Patent Nr. 5,861,153 ein Hautäquivalent, das aus einem Epidermisäquivalent auf einem Träger besteht, wobei das Epidermisäquivalent Keratinozyten und induzierte oder nicht induzierte Vorläufer von Langerhans-Zellen umfasst. Bei dem Träger kann es sich um ein Fibroblasten enthaltendes Kollagengitter handeln oder um von der Epidermis befreite Dermisabschnitte, künstliche Membranen, einen Subkutanersatz auf der Basis von Kollagen oder synthetische Materialien.

Das US-Patent Nr. 4,963,489 beschreibt ein in vitro hergestelltes Stroma-Gewebe, wobei die Stromazellen, beispielsweise Fibroblasten, ein Grundgerüst umhüllen, das aus einem biologisch verträglichen Material, beispielsweise Cellulose, besteht. Das beschriebene System läßt sich unter anderem zur Herstellung eines dreidimensionalen Hautkultursystems verwenden, wobei Keratinozyten und Melanocyten auf dem Dermisäquivalent, das heisst der dreidimensionalen Stroma-Trägermatrix, ausgebracht werden.

Das US-Patent Nr. 5,755,814 beschreibt ein Hautmodellsystem, das sich sowohl als in vitro-Testsystem als auch für therapeutische Zwecke einsetzen lässt. Das System umfasst eine dreidimensionale vernetzte Matrix von unlöslichem Kollagen mit darin enthaltenen Fibroblasten und stratifizierte Schichten differenzierter Epidermiszellen, wobei eine Epidermiszell-Schicht in direktem Kontakt zu der Oberfläche der Kollagen-Matrix steht. Die Vernetzung der Matrix kann sowohl mittels thermischer Behandlung unter Wasserentzug als auch durch chemische Mittel, beispielsweise Carbodiimid, erfolgen.

In dem US-Patent Nr. 5,882,248 ist ein Verfahren zur Bestimmung der Wirkung chemischer Substanzen oder Agenzien auf ein menschliches Hautmodellsystem gemäß US-Patent Nr. 5,755,814 beschrieben. Die Wechselwirkung zwischen dem Hautmodellsystem und den zu testenden Substanzen wird anhand der Freisetzung von Stoffen durch Zellen des Hautmodellsystems sowie der Wirkungen auf Stoffwechsel, Proliferation, Differenzierung und Reorganisation dieser Zellen bestimmt.

In der WO 95/10600 ist darüber hinaus ein Verfahren beschrieben, mit dem ein Epidermisäquivalent gewonnen werden kann. Dieses Epidermisäquivalent kann für pharmazeutische und/oder kosmetische Sonnenbräunungs-Tests verwendet werden.

Jedes der Dokumente FR-A-2 809 313, EP-A-0 702 081, EP-A-0 314 109, US 2003/107892, JP 2000 125855 und "Development of a skin model based on insoluble fibrillar collagen", GEESIN, J. of Biomed. Mat. Res. 33,1996, (seiten 1-8) offenbart ein Verfahren zur Herstellung eines Ganzhautmodells (ein Dermisäquivalentes und oder ein Epidermiäquivalentes), **dadurch gekennzeichnet, dass** man aus kollagenhaltigem Gewebe schwer lösliches Kollagen gewinnt, das schwer lösliche Kollagen mittels einer Mischvorrichtung in eine homogene Kollagensuspension überführt, die Kollagensuspension lyophilisiert und einer Vernetzung unterzieht und so eine mechanisch stabilisierte Matrix erzeugt, Fibroblasten auf diese Matrix einsät und wachsen lässt, und/oder Keratinozyten auf diese Matrix einsät und wachsen lässt und schliesslich die in und auf der Matrix wachsenden Fibroblasten und Keratinozyten bis zur vollständigen Ausbildung des dermalen und des epidermalen Teils des Ganzhautmodells weiter kultiviert.

Die internationale Patentanmeldung WO 01/092477 offenbart ein Verfahren zur Differenzierung und/oder Vermehrung isolierter dermaler Fibroblasten, wobei die Fibroblasten in einer dreidimensionalen gelartigen Matrix kultiviert werden und sich dort vermehren können. Diese Matrix enthält neben den zu kultivierenden Fibroblasten ein aus einer Kollagenlösung konstituiertes Gerüst aus menschlichem oder tierischem Kollagen, also gewebetypische Matrixproteine. Durch das Verfahren soll ein organoides in vitro-Hautmodell erhalten werden, das aus zwei gewebetypischen Schichten, nämlich einem Dermisäquivalent und einem Epidermisäquivalent, aufgebaut ist. Das organotypische Hautmodell soll sowohl histologisch als auch funktionell weitgehend der nativen Haut entsprechen.

Bei den bekannten Hautmodellen wirkt sich nachteilig aus, dass diese zumeist nur aus einer oder mehreren epidermalen Schicht(en) aus Keratinozyten bestehen. In den Fällen, in denen eine stratifizierte Epidermis erhalten wird, werden häufig Gewebe-Explantate eingesetzt, die die Gefahr einer Kontamination mit Erregern in sich bergen, was bei einer späteren Verwendung des Hautäquivalents als Testhaut zu Ergebnisverfälschungen führen kann. Sofern die beschriebenen Hautmodelle einen Dermalteil besitzen, besteht dieser häufig aus spongiösem, quervernetztem Material, das neben Kollagen außerdem noch andere nichthauttypische Materialien enthalten kann. Sofern bei den im Stand der Technik beschriebenen Hautäquivalenten der Dermalteil nur aus Kollagen und Fibroblasten besteht, ist er einem undefinierten Schrumpfungsprozess unterworfen, der auf eine starke Schrumpfung des Kollagengels und einen Flüssigkeitsaustritt daraus zurückzuführen ist. Dies führt dazu, dass die im Stand der Technik beschriebenen Hautäquivalente sich nur in beschränktem Maße als Testhaut definierter Größe eignen und die damit erhaltenen Ergebnisse sich nur bedingt auf native menschliche Haut übertragen lassen. Herkömmliche Hautäqivalente mit einem Dermalteil aus Kollagen und Fibroblasten sind zudem aufwendig in der Herstellung, da ein Kollagengel eingesetzt wird, das sich nur schwer verarbeiten läßt. Die Gellösung muß nämlich in der Kälte, also am besten auf Eis, angerührt werden und auch kalt pipettiert werden, da sie nur so flüssig bleibt. Anschließend wird die Gellösung erwärmt, z.B. auf 37° im Brutschrank. Nur unter diesen Bedingungen und dem richtig eingestellten pH-Wert kommt es zur Gelierung. Je wärmer die Gellösung wird, umso schwerer lässt sie sich verarbeiten. Wird das Gel zu stark erwärmt, besteht zudem die Gefahr, dass das Kollagen denaturiert und das Gel somit unbrauchbar wird.
Schließlich bleibt noch festzustellen, dass die aus dem Stand der Technik bekannten Hautmodelle hinsichtlich ihrer Hautähnlichkeit suboptimal sind. Insbesondere die Expression des bedeutsamen dermalen Differenzierungsmartcers Elastin ist in den bekannten Hautmodellen - im Gegensatz zu natürlicher Dermis - nicht zu beobachten.

Das der vorliegende Erfindung zu Grunde liegende technische Problem besteht also darin, ein möglichst weitgehend nativer menschlicher Haut entsprechendes humanes in vitro-Vollhautmodell sowie Verfahren und Mittel zu dessen Herstellung bereit zu stellen, das sowohl eine Epidermisschicht als auch eine Dermisschicht besitzt, das die oben genannten Nachteile des Standes der Technik überwindet und sich als Testhaut beispielsweise zur Untersuchung pharmakologischer und kosmetischer Wirkungen sowie zur Hautverträglichkeitsprüfung einsetzen lässt.

Dieses Problem wird erfindungsgemäß gelöst durch ein Verfahren zur Herstellung eines Ganzhautmodells, das dadurch gekennzeichnet ist, dass man
a) aus kollagenhaltigem Gewebe schwer lösliches Kollagen gewinnt,
b) das schwer lösliche Kollagen mittels einer Mischvorrichtung in eine homogene Kollagensuspension überführt,
c) die Kollagensuspension lyophilisiert und so eine erste Matrix A erzeugt,
d) die erste Matrix A einer Vernetzung unterzieht und so eine zweite, mechanisch stabilisierte Matrix B erzeugt,
e) Fibroblasten auf die zweite Matrix B einsät und wachsen läßt,
f) Keratinozyten auf die zweite Matrix B einsät und wachsen läßt und schließlich
g) die in und auf der Matrix B wachsenden Fibroblasten und Keratinozyten bis zur vollständigen Ausbildung des dermalen und des epidermalen Teils des Ganzhautmodells weiter kultiviert,
wobei der Lyophilisierungsprozess in Schritt c) mit einer Abkühlrate von 10 °C bis 30 °C pro Stunde durchgeführt wird.

Vorteilhafterweise läßt sich die in Schritt b) erhaltene Kollagensuspension - ganz im Gegensatz zu den im Stand der Technik eingesetzten Kollagengelen - bei Raumtemperatur problemlos gießen und pipettieren.

Das erfindungsgemäß erhältliche Ganzhautmodell läßt sich im Vergleich zu Modellen aus dem Stand der Technik gut handhaben, da es besonders robust ist. Außerdem haftet das erfindungsgemäß erhältliche Ganzhautmodell gut am Boden der Kulturgefäße, so dass es nicht zur vorzeitigen Ablösung des noch nicht vollständig ausgebildeten Hautmodells kommt, wodurch das Modell unbrauchbar würde.

Im Zusammenhang mit der vorliegenden Erfindung bedeutet der Begriff "Kultivieren" ein, vorzugsweise in vitro stattfindendes, Aufrechterhalten der Lebensfunktionen von Zellen, beispielsweise Fibroblasten oder Keratinozyten, in einer geeigneten Umgebung, beispielsweise unter Zu- und Abfuhr von Stoffwechseledukten und -produkten, insbesondere auch eine Vermehrung der Zellen.

Im Zusammenhang mit der vorliegenden Erfindung werden unter Fibroblasten natürlicherweise vorkommende, insbesondere in der Dermis vorkommende Fibroblasten, gentechnisch veränderte Fibroblasten oder aber aus Spontanmutationen hervorgegangene Fibroblasten oder deren Vorläufer verstanden. Die Fibroblasten können tierischer oder menschlicher Herkunft sein.

Unter "schwer löslichem Kollagen" wird erfindungsgemäß grobfaseriges Kollagen verstanden, das im wässrigen Medium über mehrere Stunden hinweg keine sichtbare oder nur geringe Quellung bzw. keine oder nur geringe Gelbildung, insbesondere keine Gelbildung, zeigt. Schwer lösliches Kollagen wird vorzugsweise aus den Sehnen von Tieren, insbesondere von Säugetieren, bevorzugt von Pferden, Schweinen oder Rindern, ganz besonders bevorzugt aus den Achillessehnen oderder Haut von Rindern, insbesondere aus den Achillissehnen von Rindern, gewonnen.

Die Überführung in eine homogene Kollagensuspension kann mit oder in jeder aus fachmännischer Sicht geeigneten Mischapparatur erfolgen, vorzugsweise in einem statischen Mischer oder mit einem Ultra Turrax.

Die Kollagensuspension wird nun in Behältnisse gegeben, deren Dimensionen denen der gewünschten Matrices entsprechen, z. B. in die Kavitäten einer Mikrotiterplatte.

Vor dem Befüllen der Behältnisse, z. B. der Kavitäten einer Mikrotiterplatte mit der Kollagensuspension können die Gefäße mit verschiedenen Agenzien beschichtet werden, um im folgenden die Haftung der lyophilisierten Kollagenmatrix an die Gefäßwand zu verbessern. Geeignete Agenzien sind beispielsweise Kollagen, Gelatine, Polylysin, Fibrin bzw. Fibrinogen/Thrombin oder Fibronectin. Hierbei benetzt man zunächst die inneren Gefäßwandungen mit Lösungen oder Suspensionen der Agenzien, trocknet die Gefäße, so dass sich eine Schicht der Agenzien auf der inneren Gefäßfläche bildet und füllt dann erst die Kollagensuspension ein.

Die Lyophilisierung (Gefriertrocknung) in Schritt c) erfolgt in diesen Behältnissen. Wenn das Einfrieren mit geringer Abkühlgeschwindigkeit erfolgt, so werden große Eiskristalle erhalten, die Konzentrationsunterschiede und Entmischung im Produkt bewirken. Durch ein langsames, ausgeprägtes Eiskristallwachstum entstehen besonders große Eiskristalle, die, wenn ein Temperaturgradient beim Einfrieren vorgelegen hat, in Richtung des Gradienten ausgerichtet sind. In diesem Fall werden Produkte erhalten, die von einer großlumigen Säulen- bzw. Kaminstruktur durchzogen sind. Allerdings ist bei diesen groß gewachsenen Strukturen die spezifische Oberfläche verkleinert, so dass das Rekonstitutionsverhalten negativ beeinflußt sein kann.

Aus diesem Grunde wird üblicherweise bei der Gefriertrocknung biologischen Materials darauf geachtet, eine möglichst schnelle Abkühlung zu bewirken.

Überraschenderweise hat die Anmelderin jedoch festgestellt, dass eine langsame Abkühlung der Kollagensuspension während des Lyophilisierungsprozesses vorteilhafte Auswirkungen auf die Beschaffenheit der Matrices A und B hat. Erfindungsgemäß bevorzugt ist eine Abkühlrate von 10 °C bis 30 °C pro Stunde, besonders bevorzugt 15 °C bis 25 °C pro Stunde, ganz besonders bevorzugt von 18 °C bis 23 °C und noch stärker bevorzugt von 20 °C bis 22 °C.

Solche bevorzugt erhältlichen Lyophilisate weisen ein Häutchen an der Produktoberfläche auf.

Vorteilhafterweise bildet dieses Häutchen an der Oberfläche der lyophilisierten Matrix Poren aus, die für die nachfolgende Besiedelung mit Fibroblasten besonders gute Bedingungen bieten, da sie ein langsames Einwandern der Fibroblasten in die Matrix fördern. Schnellere Abkühlungsraten während des Lyophilisationsprozesses führen hingegen häufig zu einer offenporigen Matrix mit "Kratern" in der Oberfläche, die die Fibroblasten in der gegebenen Kultivierungszeit nicht komplett mit neusynthetisierter extrazellulärer Matrix ausfüllen können. Ausgesäte Keratinozyten können in diese Krater hineinfahren, in ihnen aggregieren und ausdifferenzieren, wodurch die Schichtung und damit die Differenzierung des Hautmodells empfindlich gestört werden kann.

Die Vernetzung in Schritt d) kann mit Hilfe jedes dem Fachmann geeignet scheinenden physikalischen oder chemischen Vernetzungsverfahrens erfolgen. Überraschenderweise hat die Anmelderin gefunden, dass sich Glutaraldehyd trotz seiner cytotoxischen und apoptotischen Eigenschaften bevorzugt als Vernetzer für das erfindungsgemäße Verfahren eignet. Die Vernetzung kann jedoch auch durch physikalische Verfahren wie UV-Bestrahlung und dehydrothermale Vernetzung (DHT) erfolgen.
Als chemische Vernetzungsreagenzien sind erfindungsgemäß bifunktionale Substanzen geeignet, deren Gruppen mit den Aminogruppen der Lysin- und Hydroxylysinresten auf verschiedenen Polypeptidketten der Kollagenfasern reagieren. Weiterhin kann auch die Aktivierung der Carboxylgruppen der freien Glutamin- und Asparginsäurereste, gefolgt von einer Reaktion mit den Aminogruppen einer anderen Polypeptidkette, zu einer erfindungsgemäßen Vernetzung der Kollagenfasern führen.
Eine Vernetzung zwischen zwei Kollagenfasern kann erfindungsgemäß auch durch eine Reaktion der Aminogruppen mit Diisocyanaten oder durch die Ausbildung von Acylaziden erfolgen.
Gängig und im Sinne der vorliegenden Erfindung einsetzbar ist auch die Vernetzung mit Carbodiimide wie z.B. EDC (1-Ethyl-3-(3-dimethyl aminopropyl)-carbodiimid) unter Beteiligung von N-Hydroxysuccinimid.
Erfindungsgemäß sind auch Vernetzungsreaktionen mit homo-bifunktionalen Vernetzungsreagenzien, die mit Aminogruppen reagieren möglich, beispielsweise mit p-Benzochinon, Dimethyladipimidat, Dimethylpimelinidat, Dimethylsuberimidat, 1,4-Phenylendiisothiocyanat, Polyoxyethylen-bis (imidazolyl carbonyl), bis[Polyoxyethylen-bis(imidazolyl-carbonyl)] und Suberinsäure-bis(N-hydroxysuccinimidester); ebenso wie der Einsatz eines biologischen Vernetzungsagens, insbesondere mit Enzymen, bevorzugt Transglutaminase, das Peptidketten miteinander verknüpfen kann, oder Lysyl-Oxidasen (EC 1.4.3.13).
Weitere Vemetzungsmöglichkeiten sind beispielsweise in der EP-A2-0898973 aufgeführt.

Die Gewinnung und Kultivierung der Fibroblasten und Keratinozyten erfolgt nach der Fachwelt bekannten Verfahren, die gemäß den gewünschten Eigenschaften des herzustellenden Hautmodells angepasst werden können.

Erfindungsgemäß ist darüber hinaus in einer bevorzugten Ausführung vorgesehen, dass vor, während oder nach der Aussaat der Keratinozyten in Schritt f) auch andere Zelltypen und/oder andere Zellen anderer Gewebetypen humanen als auch tierischen Ursprungs, z. B. von Säugetieren, und/oder deren Vorläuferzellen auf der Matrix ausgesät werden können, zum Beispiel Melanocyten, Macrophagen, Monocyten, Leukocyten, Plasmazellen, neuronale Zellen, Adipocyten, induzierte und nicht induzierte Vorläuferzellen von Langerhans-Zellen, Langerhans-Zellen sowie andere Immunzellen, Endothelzellen, Zellen aus Tumoren der Haut bzw. hautassoziierter Zellen, insbesondere Sebocyten bzw. Talgdrüsengewebe oder Talgdrüsenexplantate, Zellen der Schweißdrüsen bzw. Schweißdrüsengewebe oder Schweißdrüsenexplantate, Haarfollikelzellen oder Haarfollikelexplantate; sowie Zellen aus Tumoren anderer Organe bzw. aus Metastasen. Es können auch Stammzellen unterschiedlicher Herkunft, gewebespezifische Stammzellen, embryonale und/oder adulte Stammzellen, in das Hautmodell eingebaut werden. Mit Hilfe des erfindungsgemäßen Verfahrens wird somit ein organoides in vitro-Hautmodell erhalten, das aus zwei gewebetypischen Schichten, nämlich einem Dermisäquivalent und einem Epidermisäquivalent, aufgebaut ist. Das organotypische Hautmodell entspricht sowohl histologisch als auch funktionell weitgehend der nativen Haut.

Durch die Vernetzung der Matrix A wird erreicht, dass das auf und in der Matrix B heranwachsende Dermisäquivalent im Verlauf der Kultivierungsdauer keinem oder nur einem sehr geringen Schrumpfungsprozess unterworfen ist. Dadurch werden Hautäquivalente mit definiertem Durchmesser, einheitlicher Oberfläche und einem definierten Abschluss zum Rand des Kulturgefäßes erhalten. Durch die einheitliche Größe und einheitliche Beschaffenheit des als Testoberfläche verwendeten Vollhautmodells (Ganzhautmodells) wird erreicht, dass bei Tests von Substanzen auf pharmakologische und/oder kosmetische Effekte die Qualität der Ergebnisse gesteigert wird und die Testergebnisse reproduzierbarer werden.

Die zur Kultivierung der Fibroblasten vorgesehene vernetzte Matrix B enthält also die zu kultivierenden Fibroblasten und ein aus einer, vorzugsweise frischen, Kollagensuspension menschlichen oder tierischen Ursprungs neu konstituiertes Kollagengerüst mit einer Konzentration von etwa 5 bis 50 mg Kollagen pro ml Matrix, entspr. 0,5 % bis 5 % Kollagen. Bevorzugt ist der Bereich von 8 bis 25, entsprechend 0,8 % bis 2,5 % Kollagen, insbesondere von 8 bis 12 mg Kollagen pro ml Matrix, entsprechend 0,8 % bis 1,2 % Kollagen.

Das Kollagengerüst wird aus einer, vorzugsweise zellfreien, sauren Suspension von schwer löslichem Kollagen gewonnen, wobei die Proteinkonzentration der Suspension vorzugsweise 5 bis 15 mg/ml, entsprechend 0,5 bis 1,5 Gew.%, beträgt. Der pH-Wert der Kollagenlösung beträgt 0,1 bis 6,9, vorzugsweise 2,0 bis 5,0, bevorzugt 3,0 bis 4,5 und insbesondere 3,5 bis 4,0.

Zur Kultivierung der Fibroblasten-haltigen Matrix wird die Matrix B mit einer Lösung, enthaltend ein Zellkulturmedium (vorzugsweise DMEM-Zellkulturmedium), Puffer (beispielsweise Hepes-Puffer), Serum (vorzugsweise fötales Kälberserum (FCS), normales Kälberserum (NCS), normales Lammserum (NLS), definiertes Serum oder Serumersatzprodukte) und vorzugsweise 1-6 x 10⁵/Matrix Fibroblasten, insbesondere vorkultivierten Fibroblasten, versetzt.

Zur weiteren Stabilisierung der Matrix B kann Fibronectin und/oder Laminin, vorzugsweise humanes Fibronectin und/oder Laminin, auf die Matrix bzw. in das Kulturmedium gegeben werden. Bei Fibronectinen handelt es sich um in Fibroblasten produzierte Struktur- beziehungsweise Adhäsionsproteine, deren Funktion in vivo in der Bindung an andere Makromoleküle, beispielsweise Kollagen, und in der Anheftung von Zellen an Nachbarzellen besteht. Laminin ist ein Protein der Basalmembran, an das Zellen adhärieren können. Durch die Zugabe von Fibronektinen und/oder Laminin zur Fibroblasten-Kollagenmatrix wird also die Bindung der Fibroblasten sowohl an Kollagen als auch untereinander begünstigt. Beide Proteine können entweder direkt in die Matrix eingearbeitet werden bei deren Herstellung oder nach dem Vernetzungsschritt der Matrix in gelöster Form, z.B. in Kulturmedium gelöst, zugegeben werden. Dies kann vor oder parallel zur Zellaussaat erfolgen.

Die anschließende Kultivierung der Fibroblasten in der Kollagenmatrix erfolgt vorzugsweise in Submers-Kultur. Im Zusammenhang mit der vorliegenden Erfindung wird unter einer "Submers-Kuttivierung" oder einer "Submers-Kultur" ein Verfahren zur Kultivierung von Zellen verstanden, wobei die Zellen mit einer Nährlösung bedeckt sind. Die Fibroblasten enthaltende Matrix B wird also bevorzugtermaßen mit Zellkulturmedium überschichtet und im Temperaturbereich von etwa 30 °C bis etwa 40 °C inkubiert.

Zusätzlich zu der beschriebenen Methode zur Kultivierung eines erfindungsgemäß erhaltenen Vollhautmodells kann das Modell durch Veränderungen der Kultivierungsbedingungen, z.B. durch Mediumkomponenten dahingehend optimiert werden, dass es eine bessere Barrierefunktion erhält, die der in vivo-Situation noch näher kommt. Diese ist wichtig z. B. für die Durchführung von Penetrationsstudien, aber auch für Herstellung von Produkten, die die Barrierefunktion der Haut verbessern. Darüber hinaus gibt es medizinisch relevante Störungen der Barrierefunktion, sei es durch Umweltfaktoren (Kontakt mit Detergenzien) oder genetisch bedingt. Hautmodelle mit optimierter Barrierefunktion wären hier wichtig.
Die Verbesserung der Barrierefunktion kann durch veränderte Kulturbedingungen (z.B. Luftfeuchte, Temperatur), durch chemische Komponenten im Medium (Ceramide, Vitamin C) oder durch genetisch veränderte Keratinozyten erreicht werden.
Die Veränderung der Barrierefunktion kann durch die Messung der elektrischen Kapazität der Oberfläche (surface electrical capacitance) gemessen werden.
Ausführliche Angaben zur Verbesserung der Barrierefunktion finden sich beispielsweise in der US-A1-20020168768.

Zur Verbesserung des Fibroblastenwachstums im Dermisäquivalent können dem Kulturmedium Faktoren zugefügt werden, die im Falle einer Kokultivierung von Fibroblasten und Keratinozyten von den Keratinozyten freigesetzt werden. Beispielsweise kann der konditionierte Kulturüberstand (d.h. der Mediumüberstand von kultivierten Keratinozyten und/oder Kokulturen von Keratinozyten mit anderen Zelltypen, wie z.B. Endothelzellen, Immunzellen oder Fibroblasten) verwendet werden.

In einer weiteren vorteilhaften Ausführungsform der Erfindung werden die Fibroblasten in der Matrix B, wie vorstehend beschrieben, so kultiviert, dass anschließend ein Dermisäquivalent gewonnen werden kann. Dies geschieht vorzugsweise durch ein Verfahren zur Herstellung eines Dermisäquivalentes, das dadurch gekennzeichnet ist, dass man
a. aus kollagenhaltigem Gewebe schwer lösliches Kollagen gewinnt,
b. das schwer lösliche Kollagen mittels einer Mischvorrichtung in eine homogene Kollagensuspension überführt,
c. die Kollagensuspension lyophilisiert und so eine erste Matrix A erzeugt,
d. die erste Matrix A einer Vernetzung unterzieht und so eine zweite, mechanisch stabilisierte Matrix B erzeugt,
e. Fibroblasten auf die zweite Matrix B einsät und wachsen läßt und schließlich
f. die in und auf der Matrix B wachsenden Fibroblasten bis zur vollständigen Ausbildung des Dermisäquivalentes weiter kultiviert.

Im Zusammenhang mit der vorliegenden Erfindung wird unter einem "Dermisäquivalent" eine Bindegewebe-artige Schicht aus Kollagen und Fibroblasten verstanden, die weitgehend der nativen Dermis entspricht.

Das so erhaltene Dermisäquivalent kann für Screening- und Diagnoseverfahren verwendet werden, insbesondere zur Untersuchung der Wirkungen chemischer Substanzen,Pflanzenextrakten und Metallen, beispielsweise potentieller Arzneimittel oder Bestandteile von Kosmetika, oder anderer Agenzien (physikalische Größen), wie Licht oder Wärme, Radioaktivität, Schall, elektomagnetische Strahlung, elektrische Felder und auch zur Untersuchung der Phototoxizität, also der schädigenden Wirkung von Licht unterschiedlicher Wellenlänge, auf Zellstrukturen. Das erfindungsgemäß hergestellte Dermisäquivalent kann auch zur Untersuchung der Wundheilung eingesetzt werden. Es eignet sich auch zur Untersuchung der Wirkung von Gasen, Aerosolen, Rauch, Stäuben auf die Zellstrukturen bzw. den Stoffwechsel oder die Genexpression.
Im Zusammenhang mit der vorliegenden Erfindung werden unter dem Begriff "Agens" oder "Agenzien" insbesondere auf die Haut oder Hautzellen wirkende physikalische Mittel wie Licht, Wärme, oder ähnliches, verstanden. Die Erfindung betrifft daher auch Screening- und Diagnoseverfahren unter Verwendung der erfindungsgemäß hergestellten Dermisäquivalente.

Eine bevorzugte Ausführung der Erfindung umfasst die Behandlung des Dermisäquivalents in An- und Abwesenheit der zu untersuchenden Substanz und/oder des zu untersuchenden Agens und den Vergleich der beobachteten Auswirkungen auf die Zellen oder Zellbestandteile des Dermisäquivalents.

Eine weitere bevorzugte Ausführung der Erfindung umfasst ein Verfahren zur Untersuchung der Penetration von Substanzen unter Verwendung des erfindungsgemäß hergestellten Dermisäquivalents und unter Verwendung eines erfindungsgemäßen Ganzhautäquivalents, das aus einem Dermisäquivalent und einem Epidermisäquivalent besteht.

Eine besonders bevorzugte Ausführungsform der Erfindung betrifft auch ein vorgenanntes Verfahren zur Kultivierung dermaler Fibroblasten und epidermaler Keratinozyten in einer Matrix zur Herstellung eines aus Dermisäquivalent und Epidermisäquivalent bestehenden Ganzhautäquivalentes. Dabei werden ein bis drei Wochen, vorzugsweise 10 bis 14 Tage, nach der vorstehend beschriebenen Herstellung und Inkubation der Fibroblasten enthaltenden Kollagen-Matrix Keratinozyten auf die Matrix ausgesät.

Im Zusammenhang mit der vorliegenden Erfindung werden unter "Keratinozyten" Zellen der Epidermis, die verhornendes Plattenepithel bilden, gentechnisch veränderte Keratinozyten oder aus Spontanmutationen hervorgegangene Keratinozyten oder deren Vorläufer verstanden, die tierischer oder menschlicher Herkunft sein können. Da die Ausbildung einer gut differenzierten Epidermis mit intakter Verhornung in starkem Maße vom Anteil basaler Stammzellen in den verwendeten Keratinozyten abhängt, kann es sich bei den gemäß Schritt f) des erfindungsgemäßen Verfahrens auf die Matrix ausgesäten Keratinozyten um weitgehend undifferenzierte Keratinozyten-Stammzellen aus humanem Biopsiegewebe handeln. Es ist aber auch möglich, Zelllinien oder bestimmte differenzierte Zellen einzusetzen. Alternativ zu den normalen Haut-Keratinozyten können auch Schleimhautkeratinocyten oder Darmepithelzellen auf die Matrix aufgebracht werden:
Vorzugsweise handelt es sich dabei um vorkultivierte Zellen, besonders bevorzugt um Keratinozyten in der ersten oder in der zweiten Zellpassage, es ist aber auch der Einsatz von Zellen aus höheren Passagen möglich..

Die Aussaat der Keratinozyten auf die Matrix erfolgt vorzugsweise in einem Zellkulturmedium, besonders bevorzugt in DMEM/F12-Medium das etwa 1 bis 30 % fötales Kälberserum, NCS, definiertes Serum oder Serumersatzprodukte und verschiedene Additive in wechselnden Konzentrationen enthält, die die Proliferation und Differenzierung der Zellen fördern. Anschließend wird die Matrix bevorzugtermaßen mit DMEM-Medium, enthaltend insbesondere EGF aus der Maus oder auch vergleichbare Präparate aus anderen Tieren, epidermalen Wachstumsfaktor (hEGF) (z. B. in einer Konzentration von 0,2 µg/l Medium), und beispielsweise 0,8 mM CaCl₂, überschichtet und einer vorzugsweise 1 - bis 10-tägigen, vorzugsweise 3- bis 7-tägigen Submers-Kultivierung unterworfen.

Eine vollständige Differenzierung der Keratinozytenschichten wird beispielsweise durch eine Airlift-Kultur in DMEM ohne hEGF und BPE (bovine pituitary extract) erreicht. Im Zusammenhang mit der vorliegenden Erfindung wird unter einer "Airlift-Kultur" eine Kultur verstanden, wobei die Höhe des Nährmedienspiegels genau auf die Höhe der Matrix abgestimmt ist, während die Keratinozyten oder durch die Keratinozyten gebildeten Zellschichten über dem Nährmedienspiegel liegen und vom Nährmedium nicht bedeckt werden, das heisst die Kultivierung erfolgt an der Grenzschicht Luft-Nährmedium, wobei die Versorgung der Kulturen von unten her erfolgt. Dazu werden z. B. die Hautmodelle aus einer Mikrotiterplatte gehoben und auf Filterpapiere gesetzt, die auf metallenen Abstandshaltern in Petrischalen ruhen. Das Medium wird in den Petrischalen so hoch eingefüllt, dass es das Filterpapier nicht vollständig bedeckt, dass aber ein Flüssigkeitskragen um die Basis der Hautmodelle vorhanden ist (Air-Liquid-Interface). Der Zeitraum der Airlift-Kultur kann durch den Fachmann in gewünschter Weise variiert werden. Typischerweise beträgt er etwa 1 bis 4 Wochen. Während dieses Zeitraumes entwickelt sich ein hauttypisches, aus Dermisäquivalent und Epidermisäquivalent bestehendes in vitro-Vollhautmodell.

Das erfindungsgemäße Verfahren zur Herstellung eines in vitro-Ganzhautrnodells kann vorteilhafterweise so modifiziert werden, dass vor, während oder nach der Aussaat von Keratinozyten weitere Zelltypen, wie Melanocyten, Macrophagen, Monocyten, Leukocyten, Plasmazellen, neuronale Zellen, Adipocyten, induzierte und nicht induzierte Vorläuferzellen von Langerhans-Zellen, Langerhans-Zellen andere Immunzellen, Endothelzellen sowie Zellen aus Tumoren der Haut bzw. hautassoziierter Zellen auf der Matrix ausgesät und weiter kultiviert werden können. Die genannten Zellen können sowohl humanen als auch tierischen Ursprungs sein.

Die Erfindung betrifft daher auch ein hauttypisches in vitro-Vollhautmodell, insbesondere ein humanes in vitro-Vollhautmodell, das nach dem erfindungsgemäßen Verfahren und einem gegebenenfalls anschließenden und/oder vorausgehenden Kultivierungsverfahren herkömmlicher Art hergestellt wurde und das im epidermalen Anteil mindestens eine proliferative Zellschicht, einige differenzierende Zellschichten und mindestens eine verhornte Zellschicht umfasst, wobei das Epidermisäquivalent Stratum basale, Stratum spinosum, Stratum granulosum und Stratum corneum umfasst und wobei zwischen dem Dermisäquivalent und dem Epidermisäquivalent eine Basalmembran, bestehend aus den charakteristischen BM-Proteinen wie beispielsweise Laminin und Kollagen IV enthalten ist und wobei darüber hinaus hauttypische Proteine wie Transglutaminase, Involucrin, Kollagen IV, Laminin, Filaggrin, Fibronectin, Ki-67, Cytokeratin 10 und insbesondere Elastin exprimiert werden.

Der Stressmarker Cytokeratin 6 wird hierbei jedoch nur in geringem Umfang exprimiert, was darauf hindeutet, das sich die Zellen in dem erfindungsgemäßen in vitro-Vollhautmodell in einem relativ stressarmen Zustand befinden. Starke CK6 - Expression findet man in der Haut z.B. während der Wundheilung bzw. Regeneration.

Das proliferative Verhalten sowie die Differenzierung der dermalen und epidermalen Zellen kann durch das Anlegen von elektrischen bzw. elektromagnetischen Feldern an das Dermismodell oder Vollhautmodell während der Kultivierungsphase modifiziert werden.

Aufgrund der Komplexität des hergestellten Hautmodells kann dieses gezielt für verschiedene Fragestellungen der chemisch-pharmazeutischen Industrie und der kosmetischen Industrie verwendet werden. Insbesondere eignet sich das erfindungsgemäß hergestellte Hautäquivalent zur Produktprüfung, beispielsweise im Hinblick auf Wirksamkeit, unerwünschte Nebenwirkungen, beispielsweise Reiz-, Toxizitäts- und Entzündungswirkungen oder allergieauslösende Wirkungen, oder Verträglichkeit von Substanzen. Dabei kann es sich um Substanzen handeln, die potentielle Verwendung als Arzneimittel, insbesondere als Dermatika, finden sollen, oder um Substanzen, die Bestandteil von Kosmetika sind, oder auch um Konsumgüter, die mit der Haut in Berührung kommen, wie z.B. Waschmittel etc.

Das erfindungsgemäß hergestellte Hautäquivalent kann beispielsweise auch für Studien zur Resorption, zum Transport und/oder zur Penetration von Substanzen verwendet werden. Darüber hinaus eignet es sich auch zur Untersuchung anderer Agenzien (physikalische Größen), wie Licht oder Wärme, Radioaktivität, Schall, elektomagnetische Strahlung, elektrische Felder beispielsweise zur Untersuchung der Phototoxizität, also der schädigenden Wirkung von Licht unterschiedlicher Wellenlänge, auf Zellstrukturen. Das erfindungsgemäß hergestellte Hautäquivalent kann auch zur Untersuchung der Wundheilung eingesetzt werden. Es eignet sich auch zur Untersuchung der Wirkung von Gasen, Aerosolen, Rauch, Stäuben auf die Zellstrukturen bzw. den Stoffwechsel oder die Genexpression.

Die Wirkungen von Substanzen oder Agenzien auf menschliche Haut lassen sich beispielsweise anhand der Freisetzung von Stoffen, beispielsweise Cytoki-nen oder Mediatoren, durch Zellen des humanen oder tierischen Hautmodellsystems, sowie der Wirkungen auf Genexpression, Stoffwechsel, Proliferation, Differenzierung und Reorganisation dieser Zellen ermitteln. Unter Verwendung von Verfahren zur Quantifizierung der Zellschädigung, insbesondere unter Verwendung eines Vitalfarbstoffes, wie eines Tetrazoliumderivates, können beispielsweise cytotoxische Wirkungen auf Hautzellen nachgewiesen werden. Die Tests von Substanzen oder Agenzien an dem erfindungsgemäßen humanen Hautäquivalent können sowohl histologische Verfahren als auch immunologische und/oder molekularbiologische Verfahren umfassen.

Eine bevorzugte Ausführungsform der Erfindung umfasst daher Verfahren zur Untersuchung der Wirkung, insbesondere der pharmakologischen Wirkungen, von Substanzen oder Agenzien auf menschliche Haut unter Verwendung des erfindungsgemäß hergestellten humanen Hautäquivalentes. In einer besonders bevorzugten Ausführungsform wird dabei ein XTT-Tetrazoliumreduktionstest (EZ4U-Test) oder Alamar Blue (Resazurinreduktionstest, Fa. Promega) durchgeführt. EZ4U ist ein nicht-toxisches wasserlösliches gelbes Tetrazoliumsalz, das von lebenden Zellen zu intensiv gefärbten Formazanen reduziert werden kann. Die Reduktion erfordert intakte Mitochondrien und der Test kann daher zum Nachweis der Vitalität von Zellen eingesetzt werden. Erfindungsgemäß einsetzbare Vitalitätsassays, die auch auf der Basis von Tetrazoliumverbindungen ruhen, sind z. B. der MTT-, MTS- bzw. der WST-1-Test. Diese werden als Testkits u. a. von der Firma Roche angeboten. Der Nachweis der Vitalität der Zellen kann auch über die Freisetzung der Lactatdehydrogenase (LDH) aus Zellen mit geschädigter Zellmembran erfolgen. Mit Trypanblau können Zellen mit geschädigter Zellmembran selektiv angefärbt werden.

Eine weitere bevorzugte Ausführungsform der Erfindung umfasst ein Verfahren zur Untersuchung der Penetration von Substanzen, wobei sowohl ein erfindungsgemäß hergestelltes Dermisäquivalent als auch ein erfindungsgemäß hergestelltes Hautäquivalent mit den zu untersuchenden Substanzen behandelt werden und die bei beiden Systemen erhaltenen Ergebnisse miteinander verglichen werden.

In einer besonders bevorzugten Ausführungsform der Erfindung werden die Wirkungen chemischer Substanzen oder anderer Agenzien auf spezielle Hauttypen untersucht. Dabei werden Zellen definierter Hauttypen, beispielsweise Hauttypen mit wenig Pigmenten und/oder Hauttypen mit vielen Pigmenten, zur Etablierung erfindungsgemäßer Hautäquivalente eingesetzt und diese werden im Hinblick auf die Wirkung von Substanzen oder Agenzien getestet.

In einer weiteren besonders bevorzugten Ausführungsform der Erfindung wird das erfindungsgemäß hergestellte Hautäquivalent als Modellsystem zu Untersuchungen von Hautkrankheiten und zur Entwicklung neuer Behandlungsmöglichkeiten für Hautleiden verwendet. Beispielsweise können Zellen von Patienten mit einer bestimmten genetisch bedingten oder erworbenen Hautkrankheit verwendet werden, um daraus patientenspezifische Hautmodellsysteme zu etablieren und daran die Wirksamkeit bestimmter Therapien und/oder Medikamente zu untersuchen und zu beurteilen.

Gemäß einer bevorzugten Ausführungsform kann das erfindungsgemäß hergestellte Hautäquivalent mit Mikroorganismen, insbesondere pathogenen Mikroorganismen besiedelt werden. Insbesondere bevorzugt ist eine Besiedelung mit pathogenen oder parasitären, insbesondere auch humanpathogenen Mikroorganismen. Unter Mikroorganismen sind im erfindungsgemäßen Sinne insbesondere Pilze, Bakterien und Viren zu verstehen.
Bevorzugt sind die Mikroorganismen dabei ausgewählt aus Pilzen oder pathogenen und/oder parasitären Bakterien. Insbesondere bevorzugt sind als Pilze die Spezies der Gattung Candida, Malassezia und Trichophyton, insbesondere bevorzugt *Candida albicans, Trichophyton mentagrophytes* und *Malassezia furfur*. Als pathogene und/oder parasitäre Bakterien sind insbesondere *Staphylococcus aureus* bevorzugt.
Mit einem solchermaßen besiedelten Hautäquivalent kann sowohl der Prozess der Besiedelung, insbesondere der Infektionsprozess, durch den Mikroorganismus selbst als auch die Antwort der Haut auf diese Besiedelung untersucht werden.
Weiterhin ist es mit einem solchen Hautäquivalent möglich, die Wirkung von vor, während oder nach der Besiedelung aufgebrachten Substanzen auf die Besiedelung selbst oder auf die Auswirkungen der Besiedelung auf das Hautäquivalent zu untersuchen.

Die Erfindung betrifft auch eine vernetzte Matrix, in der die vorgenannten Kultivierungsverfahren durchgeführt werden können. Die erfindungsgemäß vorgesehene Kombination aus Matrix und darin kultivierten Fibroblasten kann, wie vorstehend beschrieben, zur Herstellung eines Dermisäquivalentes, eines Epidermisäquivalentes und/oder eines organoiden Vollhautmodells verwendet werden.

Ein weiterer Gegenstand der vorliegenden Erfindung ist ein Verfahren zur Herstellung eines Epidermismodells, das dadurch gekennzeichnet ist, dass man
a. aus kollagenhaltigem Gewebe schwer lösliches Kollagen gewinnt,
b. das schwer lösliche Kollagen mittels einer Mischvorrichtung in eine homogene Kollagensuspension überführt,
c. die Kollagensuspension lyophilisiert und so eine erste Matrix A erzeugt,
d. die erste Matrix A einer Vernetzung unterzieht und so eine zweite, mechanisch stabilisierte Matrix B erzeugt,
e. Keratinozyten in die zweite Matrix B einsät und wachsen läßt und schließlich
f. die in und auf der Matrix B wachsenden Keratinozyten bis zur vollständigen Ausbildung des Epidermismodells weiter kultiviert.

In diesem Fall werden - anders als bei dem erfindungsgemäßen Verfahren zur Herstellung des Ganzhautmodells - nur Keratinozyten auf die Matrix ausgesät, die als Gerüst bzw. Träger für das Epidermismodell fungiert, und unter solchen Bedingungen kultiviert, dass die Keratinozyten zunächst proliferieren und sich dann so differenzieren, dass eine aus allen 4 Schichten bestehende Epidermis gebildet wird. Die Matrix wird vorzugsweise mit Proteinen der extrazellulären Matrix bzw. der Basalmembran vorbehandelt, um eine bessere Adhäsion der Keratincoyten an das Matrixmaterial zu erreichen und um den Zellen die durch diese Proteine vermittelten Signale zu bieten. Beispiele für erfindungsgemäß geeignete Proteine sind Kollagen IV bzw. Kollagene anderer Typen, Fibronectin und Laminin. Das so gebildete Epidermismodell ist ein weiterer Gegenstand der vorliegenden Erfindung und kann wie das Vollhautmodell auch für vielseitige Tests eingesetzt werden.

Bevorzugte Zellkulturmedien sind DMEM (Dulbecco's Modified Eagle Medium), M199 und Ham's F12-Medium. Jedoch kann auch jedes andere beliebige Zellkulturmedium verwendet werden, welches die Kultivierung von Fibroblasten ermöglicht. Als Serum wird vorzugsweise fötales Kälberserum (FCS), aber auch NCS sowie Serumersatzprodukte verwendet und als Puffer zum Beispiel Hepes-Puffer. Der pH-Wert der Lösung aus Zellkulturmedium, Puffer und Serum beträgt in bevorzugter Ausführung 6,0 bis 8,0, beispielsweise 6,5 bis 7,5, insbesondere 7,0

Erfindungsgemäß kann das Medium weitere Faktoren, beispielsweise Hormone, Wachstumsfaktoren, Adhäsionsmittel, Antibiotika, Selektionsmittel, Enzyme und Enzyminhibitoren und ähnliche enthalten.

Zur Verbesserung des Keratinozytenwachstums im Epidermisäquivalent können dem Kulturmedium Faktoren zugefügt werden, die im Falle einer Kokultivierung von Fibroblasten und Keratinozyten von den Fibroblasten freigesetzt werden. Beispielsweise kann der konditionierte Kulturüberstand (d.h. der Mediumüberstand von kultivierten Fibroblasten und/oder Kokulturen von Fibroblasten mit anderen Zelltypen, wie z.B. Endothelzellen, Immunzellen oder Keratinozyten) verwendet werden.

Die folgenden Figuren und Beispiele erläutern die Erfindung, ohne sie jedoch darauf einzuschränken:

### Beispiel 1:

### Herstellung der Matrix A:

In ein 1000 ml Becherglas werden 700 ml vollentsalztes Wasser gegeben, das mit 875 µl konzentrierter Essigsäure (Eisessig) angesäuert wird. Mit Hilfe eines handelsüblichen Magnetrührers mit Rührfisch wird die Lösung gerührt. In die rotierende Wassersäule hinein werden 7,0 g Rindersehnenkollagen auf einmal eingerührt. Anschließend wird das Gemisch vom Rührer genommen und für 4 Stunden ohne Rühren bei Raumtemperatur belassen. Alle 30 Minuten wird der pH-Wert des Gemisches kontrolliert. Der pH sollte in einem Bereich zwischen pH 3,5 und 4 liegen. Ebenfalls alle 30 Minuten wird die Suspension für 1 Minute mit hoher Geschwindigkeit gerührt. Es muss darauf geachtet werden, dass dabei die Temperatur der Suspension nicht ansteigt. Schließlich wird die Suspension für 3 Minuten mit hoher Geschwindigkeit gerührt und hierdurch homogenisiert. Um eine Erwärmung durch den Homogenisationsprozess zu vermeiden, wird die Suspension auf Raumtemperatur gehalten. Man erhält eine milchig-trübe Kollagensuspension. Um evtl. vorhandene Luftblasen aus der Suspension zu entfernen, kann sie bei 400 UpM (= 24,73 g) für 1 Minute bei 20 °C zentrifugiert werden (Hettich Universal 16 R, Rotordurchmesser 138 mm). Die Blasen entweichen dann ohne Schaumbildung
Mit einem Dispenser wird die Suspension in die Vertiefungen einer Zellkulturplatte mit 24 Vertiefungen pipettiert. Die gefüllte Platte wird anschließend in die Hand genommen, leicht gekippt und nach allen Seiten gedreht, damit die Gellösung die Wand der Vertiefungen auch oberhalb der Matrixoberfläche benetzt. Durch diese Verfahrensweise wird die Haftung der Matrices in den Vertiefungen verbessert. Die so behandelten gefüllten Platten werden direkt in den Lyophilisator gestellt und im Gerät eingefroren.

Die in die Zellkulturplatten mit 24 Vertiefungen eingefüllte Kollagensuspension wird in einem Lyophilisator mit temperierbaren Stellflächen eingefroren und anschließend bei Unterdruck getrocknet.

Ausgehend von einer Temperatur der Gellösung von 20°C beträgt die Einfrierrate 18 °C bis 23 °C pro Stunde. Der gesamte Gefriertrocknungsprozess dauert ca. 20-27 Stunden.

### Beispiel 2: .

### Vernetzung der Matrix A zur Herstellung der Matrix B

Um eine möglichst große und ebene Oberfläche der Hautmodelle zu erhalten, werden die Kollagenmatrices vor der Einsaat der Fibroblasten chemisch fixiert.
Als Fixiermittel wird Glutaraldehyd (GA) eingesetzt: C₅H₈O₂, MG = 100,12.

Auf jede Matrix von 24 Matrices A in einer Zellkulturplatte mit 24 Kavitäten wird mit einer Pipette vorsichtig Glutaraldehydlösung gegeben. Die Lösung wird langsam am inneren Rand der Vertiefung herunterlaufen gelassen, um die Matrixoberfläche nicht zu beschädigen. Die Glutaraldehydlösung benötigt mehrere Minuten, um in das Innere der Kollagenschwämme einzudringen. Die zunehmende Durchtränkung macht sich in einer Farbänderung der Matrices von rein weiß zu grau bis fahlgelb bemerkbar. Anschließend wird der Deckel auf die Zellkulturplatten gesetzt und der Rand mit Parafilm luftdicht verschlossen, um eine Verdunstung der Lösung zu vermeiden. Die behandelten Platten werden lichtgeschützt bei Raumtemperatur für 24 Stunden gelagert.

Die Vernetzung der Matrices einschließlich Waschen und Äquilibrieren kann in einem Zeitraum von vorzugsweise 5 Tagen, jedoch mindestens 4 Tagen, durchgeführt werden.

### Beispiel 3:

### Herstellung eines Dermisäquivalents

Vor der Aussaat auf die vernetzten Collagen-Matrices werden Fibroblasten einer geeigneten Passage in Zellkulturflaschen mit Fibroblastenmedium vorkultiviert. Nach Erreichen der gewünschten Zelldichte wird das Kulturmedium abgesaugt. Die Zellen werden durch Zugabe einer Trypsinlösung in die Kulturflaschen vom Gefäßboden abgelöst, mit Kulturmedium gewaschen und abzentrifugiert. Nach Bestimmung der Zellzahl wird die Zellsuspension auf eine Konzentration von 1-6x10⁵ Fibroblasten/ml eingestellt.
Aus den Vertiefungen der Mikrotiterplatte, die die mit Fibroblastenmedium äquilibrierten Matrices enthalten, wird das Medium so weit abgesaugt, das die Matrices feucht bleiben. Auf die Oberfläche der Matrices wird jeweils 1 ml Fibroblastenmedium, enthaltend jeweils 1-6x10⁵ Fibroblasten, pipettiert, ohne die Oberfläche dabei zu verletzen. Nach Beendigung der Einsaat wird der Plattendeckel aufgesetzt und die Platte waagerecht in den Inkubator gestellt. Die Kultivierung der Fibroblasten auf der Matrix erfolgt bei 37° und 5 % v/v CO₂. Das Kulturmedium wird in regelmäßigen Intervallen gewechselt. Nach 2-4 Wochen ist das Dermismodell fertig entwickelt.

### Beispiel 4:

### Herstellung eines Vollhautmodells

Vor der Aussaat auf die vernetzten Collagen-Matrices werden Fibroblasten einer geeigneten Passage in Zellkulturflaschen mit Fibroblastenmedium vorkultiviert. Nach Erreichen der gewünschten Zelldichte wird das Kulturmedium abgesaugt. Die Zellen werden durch Zugabe einer Trypsinlösung (oder einer anderen zur Ablösung adhärenter Zellen geeigneten Lösung) in die Kulturflaschen vom Gefäßboden abgelöst, mit Fibroblastenmedium gewaschen und abzentrifugiert. Nach Bestimmung der Zellzahl wird die Zellsuspension auf eine Konzentration von 1-6x10⁵ Fibroblasten/ml eingestellt.
Aus den Vertiefungen der Mikrotiterplatte, die die mit Kulturmedium äquilibrierten Matrices enthalten, wird das Medium so weit abgesaugt, dass die Oberfläche der Matrices feucht bleibt. Auf die Oberfläche der Matrices wird jeweils 1 ml Fibroblastenmedium, enthaltend jeweils 1-6x10⁵ Fibroblasten, pipettiert, ohne die Oberfläche dabei zu verletzen. Nach Beendigung der Einsaat wird der Plattendeckel aufgesetzt und die Platte waagerecht in den Inkubator gestellt. Die Kultivierung der Fibroblasten auf der Matrix erfolgt bei 37° und 5 % CO₂. Das Kulturmedium wird in regelmäßigen Intervallen gewechselt.
Nach einer Kultivierungszeit der Fibroblasten von 2-4 Wochen werden die Keratinozyten auf das Dermismodell ausgesät. Dazu werden Keratinozyten einer geeigneten Passage in Zellkulturflaschen mit Keratinozytenmedium vorkultiviert. Nach Erreichen der gewünschten Zelldichte wird das Kulturmedium abgesaugt. Die Zellen werden durch Zugabe einer Trypsinlösung in die Kulturflaschen vom Gefäßboden abgelöst, mit Kulturmedium (Keratinozytenmedium) gewaschen und abzentrifugiert. Nach Bestimmung der Zellzahl wird die Zellsuspension auf eine Konzentration von 1-6x10⁵ Keratinozyten/ml eingestellt. Aus den Vertiefungen der Mikrotiterplatte, die die Dermisäquivalente enthalten, wird das Medium so weit abgesaugt, dass die Oberfläche der Dermisäquivalente feucht bleibt. Auf die Oberfläche der Dermismodelle wird jeweils 1 ml Keratinozytenmedium, enthaltend jeweils 1-6x10⁵ Keratinozyten, pipettiert, ohne die Oberfläche dabei zu verletzen. Nach Beendigung der Einsaat wird der Plattendeckel aufgesetzt und die Platte waagerecht in den Inkubator gestellt (Submerskultur). Die Kultivierung der Fibroblasten und Keratinozyten auf der Matrix erfolgt 3-7 Tage bei 37° und 5 % CO₂. Das Kulturmedium wird in regelmäßigen Intervallen gewechselt.
Nach Ablauf der 3-7-tägigen Submerskultur wird das Keratinozytenmedium aus den Vertiefungen abgesaugt. Die noch unfertigen Vollhautmodelle werden aus den Vertiefungen herausgeholt und auf Filterpapiere gesetzt. Die Filterpapiere liegen auf metallenen Abstandhaltern jeweils in einer Petrischale. Nachdem die unfertigen Vollhautmodelle auf dem Filterpapier platziert sind, wird die Petrischale mit Kulturmedium (Airlift-Medium; Air-Liquid-Interface-Medium) so weit gefüllt, dass das Medium den oberen Rand des Filterpapiers erreicht und sich um die Basis der Hautmodelle verteilt. Die Oberfläche der Hautmodelle wird nicht von Kulturmedium bedeckt (Airlift-Kultur oder Air-Liquid-Interface). In Abhängigkeit vom gewünschten Differenzierungsgrad werden die Hautmodelle 1-4 Wochen in der Air-Liquid-Interface belassen. Das Kulturmedium wird in regelmäßigen Intervallen gewechselt.

Die Zusammensetzung der 3 unterschiedlichen Kulturmedien finden sich in den folgenden Tabellen:

**Fibroblasten:**

| | Endkonzentration |
|---|---|
| DMEM (Glutamax I) | |
| FCS | 10% |
| Penicillin G | 100 UI/ml |
| Gentamicin | 25 µg/ml |
| Ascorbyl-2-phosphat | 1 mM |

**Keratinozyten:**

| | Endkonzentration |
|---|---|
| DMEM (Glutamax I) | |
| HAM F 12 | |
| Fetal Clone 11 | 10% |
| EGF | 10 ng/ml |
| Hydrocortisone | 0,4 µg/ml |
| Insulin | 0,12 Ul/ml |
| Choleratoxin | 10⁻¹⁰ M |
| Tri-lodothyronine | 2*10⁻⁹ M (5 µg/ml) |
| Adenine | 2,43 µg/ml |
| Penicillin G | 100 Ul/ml |
| Gentamicin | 25 µg/ml |
| Ascorbyl-2-phosphat | 1 mM |

**Air-Liquid-Interface:**

| | Endkonzentration |
|---|---|
| DMEM (Glutamax I) | |
| HAM F 12 | |
| Hydrocortisone | 0,4 µg/ml |
| Insulin | 0,12 Ul/ml |
| Penicillin G | 100 Ul/ml |
| Gentamicin | 25 µg/ml |
| BSA | 1,6 mg/ml |
| Ascorbyl-2-phosphat | 1 mM |

### Beispiel 5:

### Nachweis von Elastin im Vollhautmodell

Fertig ausdifferenzierte Hautmodelle werden direkt aus der Air-Liquid-Interface entnommen, im Kryostaten eingefroren und geschnitten (Schnittdicke 8 µm). Die Schnitte werden für 10 Minuten in -20°C kalten Aceton fixiert und anschließen mit TBS (TRIS-buffered saline) mehrmals gewaschen. Der anti-Elastin-Antikörper (rabbit; Fa. Novotec) wird 1:40 mit TBS verdünnt, auf die Schnitte aufgetropft und für 60 Minuten auf diesen belassen. Anschließend werden die Schnitte dreimal für jeweils 5 Minuten in TBS gewaschen. Als sekundärer Antikörper wird ein goat-antirabbit-Antikörper mit Alexa-Konjugat (Fa. Molecular Probes) in einer 1:20-Verdünnung eingesetzt. Um die hohe Eigenfluoreszenz der Collagenmatrix zu blockieren, wird die Verdünnung des sekundären Antikörpers in einer O,1% Evans Blue-Lösung angesetzt. Der sekundäre Antikörper wird auf die Schnitte aufgetropft und auf diesen für 60 Minuten belassen. Danach werden sie dreimal für jeweils 5 Minuten mit TBS gewaschen. Zum Schluss werden die antikörper-markierten Schnitte in DAKO Faramount Einbettmedium eingedeckelt. Als Negativ-Kontrolle werden Schnitte ohne den ersten Antikörper inkubiert.

## Patentansprüche

1. Verfahren zur Herstellung einer Kollagenmatrix, **dadurch gekennzeichnet, dass** man
a. aus kollagenhaltigem Gewebe schwer lösliches Kollagen gewinnt,
b. das schwer lösliche Kollagen mittels einer Mischvorrichtung in eine homogene Kollagensuspension überführt,
c. die Kollagensuspension lyophilisiert und so eine erste Matrix A erzeugt,
d. die erste Matrix A einer Vernetzung unterzieht und so eine zweite, mechanisch stabilisierte Matrix B erzeugt,
wobei der Lyophilisierungsprozess in Schritt c) mit einer Abkühlrate von 10 °C bis 30 °C pro Stunde durchgeführt wird.

2. Verfahren zur Herstellung eines Dermisäquivalentes, **dadurch gekennzeichnet, dass** man
a. eine zweite, mechanisch stabilisierte Matrix B in einem Verfahren gemäß Anspruch 1 erzeugt,
b. Fibroblasten auf die zweite Matrix B einsät und wachsen lässt und schließlich
c. die in und auf der Matrix B wachsenden Fibroblasten bis zur vollständigen Ausbildung des Dermisäquivalentes weiter kultiviert.

3. Verfahren zur Herstellung eines Epidermisäguivalentes, **dadurch gekennzeichnet, dass** man
a. eine zweite, mechanisch stabilisierte Matrix B in einem Verfahren gemäß Anspruch 1 erzeugt.
b. Keratinozyten auf die zweite Matrix B einsät und wachsen lässt und schließlich
c. die in und auf der Matrix B wachsenden Keratinozyten bis zur vollständigen Ausbildung des Epidermisäquivalentes weiter kultiviert.

4. Verfahren zur Herstellung eines Ganzhautmodells, **dadurch gekennzeichnet, dass** man
a. eine zweite, mechanisch stabilisierte Matrix B in einem Verfahren gemäß Anspruch 1 erzeugt,
b. Fibroblasten auf die zweite Matrix B einsät und wachsen lässt,
c. Keratinozyten auf die zweite Matrix B einsät und wachsen lässt und schließlich
d. die in und auf der Matrix B wachsenden Fibroblasten und Keratinozyten bis zur vollständigen Ausbildung des dermalen und des epidermalen Teils des Ganzhautmodells weiter kultiviert.

5. Verfahren nach einem der Ansprüche 1 bis 4, **dadurch gekennzeichnet, dass** das es sich bei dem schwer löslichen Kollagen um ein grobfaseriges Kollagen handelt, das im wässrigen Medium über mehrere Stunden hinweg keine sichtbare oder nur geringe Quellung oder Gelbildung zeigt, vorzugsweise um schwer lösliches Kollagen aus den Sehnen von Tieren, insbesondere von Säugetieren, bevorzugt von Pferden, Schweinen oder Rindern, ganz besonders bevorzugt aus den Achillessehnen von Rindern.

6. Verfahren nach einem der vorhergehenden Ansprüche, **dadurch gekennzeichnet, dass** der Lyophilisierungsprozeß in Schritt c) mit einer Abkühlrate von 15 °C bis 25 °C pro Stunde, ganz besonders bevorzugt von 18 °C bis 23 °C und noch stärker bevorzugt von 20 °C bis 22 °C pro Stunde durchgeführt wird.

7. Ganzhautmodell, erhältlich nach einem Verfahren gemäß Anspruch 4.

8. Ganzhautmodell nach Anspruch 7, **dadurch gekennzeichnet, dass** hauttypische Proteine, insbesondere Elastin, exprimiert werden.

9. Ganzhautmodell nach Anspruch 7 oder 8, **dadurch gekennzeichnet, dass** es mit Mikroorganismen, insbesondere pathogenen Mikroorganismen besiedelt wird.

10. Ganzhautmodell nach Anspruch 9, **dadurch gekennzeichnet, dass** die Mikroorganismen ausgewählt sind aus *Staphylococcus aureus* sowie Spezies der Gattung Candida, Malassezia und Trichophyton.

11. Kollagenmatrix, erhältlich nach einem Verfahren gemäß Anspruch 1.

12. Dermisäquivalent, erhältlich nach einem Verfahren gemäß Anspruch 2.

13. Epidermisäquivalent, erhältlich nach einem Verfahren gemäß Anspruch 3.

14. Verwendung einer Kollagenmatrix gemäß Anspruch 11 zur Herstellung eines Dermisäquivalentes, eines Epidermisäquivalentes oder eines Ganzhautmodells.

15. Verwendung eines Ganzhautmodells gemäß Anspruch 7 bis 10, eines ' Dermisäquivalentes gemäß Anspruch 12 oder eines Epidermisäquivalentes gemäß Anspruch 13 zur Produktprüfung, insbesondere im Hinblick auf Wirksamkeit, unerwünschte Nebenwirkungen, Reiz-, Toxizitäts- und Entzündungswirkungen oder allergieauslösende Wirkungen, oder Verträglichkeit von Substanzen.

## Claims

1. A method for preparing a collagen matrix, **characterized in that**
a. hardly soluble collagen is extracted from collagen-containing tissue,
b. the hardly soluble collagen is converted by means of a mixing device into a homogeneous collagen suspension,
c. the collagen suspension is freeze-dried and thus produces a first matrix A,
d. the first matrix A is subject to cross-linking and thus produces a second mechanically stabilized matrix B,
wherein the freeze-drying process in step c) is carried out with a cooling rate of 10°C to 30°C per hour.

2. A method for preparing a dermis equivalent, **characterized in that**
a. a second, mechanically stabilized matrix B is produced in a method according to claim 1,
b. fibroblasts are sown on the second matrix B and left to grow and finally
c. the fibroblasts growing in and on the matrix B are further cultivated until complete formation of the dermis equivalent.

3. A method for preparing an epidermis equivalent, **characterized in that**
a. a second, mechanically stabilized matrix B is produced in a method according to claim 1,
b. keratinocytes are sown on the second matrix B and left to grow and finally
c. the keratinocytes growing in and on the matrix B are further cultivated until complete formation of the epidermis equivalent.

4. A method for preparing a complete skin model, **characterized in that**
a. a second mechanically stabilized matrix B is produced in a method according to claim 1,
b. fibroblasts are sown on the second matrix B and left to grow,
c. keratinocytes are sown on the second matrix B and left to grow and finally
d. the fibroblast and keratinocytes growing in and on the matrix B are further cultivated until complete formation of the dermal and epidermal parts of the complete skin model.

5. The method according to any of claims 1 to 4, **characterized in that**, as for the hardly soluble collagen, this is a collagen with coarse fibers, which in a aqueous medium shows no visible or only slight swelling or gelling over several hours, preferably a hardly soluble collagen from tendons of animals, in particular of mammals, preferably of horses, pigs or cattle, most preferably from the Achilles tendons of cattle.

6. The method according to any of the preceding claims, **characterized in that** the freeze-drying process in step c) is carried out with a cooling rate from 15°C to 25°C per hour, most preferably from 18°C to 23°C and even more preferably from 20°C to 22°C per hour.

7. A complete skin model, which may be obtained from a method according to claim 4.

8. The complete skin model according to claim 7, **characterized in that** skin typical proteins, in particular elastin, are expressed.

9. The complete skin model according to claim 7 or 8, **characterized in that** it is populated with microorganisms, in particular pathogenic microorganisms.

10. The complete skin model according to claim 9, **characterized in that** the microorganisms are selected from *Staphylococcus aureus* as well as species from the genus *Candida, Malassezia* and *Trichophyton*.

11. A collagen matrix, which may be obtained from a method according to claim 1.

12. A dermis equivalent, which may be obtained from a method according to claim 2.

13. An epidermis equivalent, which may be obtained from a method according to claim 3.

14. Use of a collagen matrix according to claim 11 for preparing a dermis equivalent, an epidermis equivalent or a complete skin model.

15. Use of a complete skin model according to claims 7 to 10, of a dermis equivalent according to claim 12 or an epidermis equivalent according to claim 13 for testing products, in particular with respect to effectiveness, undesired secondary effects, irritation, toxicity and inflammatory effects, or allergy triggering effects, or compatibility of substances.

## Revendications

1. Procédé pour la production d'une matrice de collagène, **caractérisé en ce que**
a. on extrait du collagène peu soluble d'un tissu contenant du collagène,
b. on transforme le collagène peu soluble au moyen d'un dispositif de mélange en une suspension homogène de collagène,
c. on lyophilise la suspension de collagène et on génère ainsi une première matrice A,
d. on soumet la première matrice A à une réticulation et on génère ainsi une deuxième matrice B mécaniquement stabilisée,
le processus de lyophilisation dans l'étape c) étant réalisé à une vitesse de refroidissement de 10°C à 30°C par heure.

2. Procédé pour la production d'un équivalent de derme, **caractérisé en ce que**
a. on génère une deuxième matrice B mécaniquement stabilisée par un procédé selon la revendication 1,
b. on ensemence des fibroblastes sur la deuxième matrice B et on les laisse croître, et finalement
c. on continue la culture des fibroblastes croissant dans et sur la matrice B jusqu'à la formation complète de l'équivalent de derme.

3. Procédé pour la production d'un équivalent d'épiderme, **caractérisé en ce que**
a. on génère une deuxième matrice B mécaniquement stabilisée par un procédé selon la revendication 1,
b. on ensemence des kératinocytes sur la deuxième matrice B et on les laisse croître, et finalement
c. on continue la culture des kératinocytes croissant dans et sur la matrice B jusqu'à la formation complète de l'équivalent d'épiderme.

4. Procédé pour la production d'un modèle de peau totale, **caractérisé en ce que**
a. on génère une deuxième matrice B mécaniquement stabilisée par un procédé selon la revendication 1,
b. on ensemence des fibroblastes sur la deuxième matrice B et on les laisse croître,
c. on ensemence des kératinocytes sur la deuxième matrice B et on les laisse croître, et finalement
d. on continue la culture des fibroblastes et des kératinocytes croissant dans et sur la matrice B jusqu'à la formation complète de la partie dermique et épidermique du modèle de peau totale.

5. Procédé selon l'une quelconque des revendications 1 à 4, **caractérisé en ce qu'**il s'agit, pour le collagène peu soluble, de collagène à grosses fibres, qui, en milieu aqueux, sur plusieurs heures, ne présente pas de gonflement ou de gélification visible ou seulement un faible gonflement et une faible gélification, de préférence de collagène peu soluble provenant de tendons d'animaux, en particulier de mammifères, de préférence de chevaux, de porcs ou de boeufs, de manière tout particulièrement préférée du tendon d'Achille de boeufs.

6. Procédé selon l'une quelconque des revendications précédentes, **caractérisé en ce que** le processus de lyophilisation dans l'étape c) est réalisé à une vitesse de refroidissement de 15°C à 25°C par heure, de manière tout particulièrement préférée de 18°C à 23°C et de manière encore plus préférée de 20°C à 22°C par heure.

7. Modèle de peau totale, pouvant être obtenu selon un procédé selon la revendication 4.

8. Modèle de peau totale selon la revendication 7, **caractérisé en ce que** des protéines caractéristiques de la peau, en particulier l'élastine, sont exprimées.

9. Modèle de peau totale selon la revendication 7 ou 8, **caractérisé en ce qu'**il est colonisé par des microorganismes, en particulier des microorganismes pathogènes.

10. Modèle de peau totale selon la revendication 9, **caractérisé en ce que** les microorganismes sont choisis parmi Staphylococcus aureus ainsi que d'espèces du genre Candida, Malassezia et Trichophyton.

11. Matrice de collagène, pouvant être obtenue selon un procédé selon la revendication 1.

12. Equivalent de derme, pouvant être obtenu selon un procédé selon la revendication 2.

13. Equivalent d'épiderme, pouvant être obtenu selon un procédé selon la revendication 3.

14. Utilisation d'une matrice de collagène selon la revendication 11 pour la préparation d'un équivalent de derme, d'un équivalent d'épiderme ou d'un modèle de peau totale.

15. Utilisation d'un modèle de peau totale selon la revendication 7 à 10, d'un équivalent de derme selon la revendication 12 ou d'un équivalent d'épiderme selon la revendication 13 pour tester des produits, en particulier en ce qui concerne l'efficacité, les effets secondaires non souhaités, les effets d'irritation, de toxicité et d'inflammation ou les effets de déclenchement d'allergies ou la compatibilité de substances.
